# EUROPEAN PATENT APPLICATION

(11) **EP 3 945 525 A1**
(43) Date of publication of application: **02.02.2022**
(21) Application number: 20187813.9
(22) Date of filing: 27.07.2020
(51) Int. Cl.: G16B 20/00, G16B 30/00, G16B 40/20

(54) **METHODS FOR IDENTIFYING CHROMOSOMAL SPATIAL INSTABILITY SUCH AS HOMOLOGOUS REPAIR DEFICIENCY IN LOW COVERAGE NEXT-GENERATION SEQUENCING DATA**

(71) Applicant: Sophia Genetics S.A., 1025 Saint Sulpice (CH)
(72) Inventor: POZZORINI, Christian, 1025 Saint-Sulpice (CH); ANDRE, Grégoire, 1025 Saint-Sulpice (CH); COLETTA, Tommaso, 1025 Saint-Sulpice (CH); XU, Zhenyu, 1025 Saint-Sulpice (CH)
(74) Representative: Wenger, Joel-Théophile

(57) **Abstract**

A genomic data analyzer may be configured to detect and characterize, with a machine learning model such as a trained convolutional neural network, the presence of a chromosomal spatial instability in a tumor sample. The genomic data analyzer may use whole genome sequencing reads as input data even at low depth coverage in a high throughput sequencing workflow as may be routinely employed in a diversity of clinical oncology setups. The genomic data analyzer may spatially arrange the read data coverage from chromosome arms or full chromosomes by aligning them according to their centromeric or telomeric bin positions to form a multidimensional array. The trained machine learning model may process the spatially arranged coverage array to determine whether a chromosomal spatial instability (CSI) such as for instance a chromosomal instability causing a homologous repair or recombination deficiency (HRD) is present in the tumor sample. The latter indication may guide the choice of a preferred anticancer treatment for the tumor.

## Description

### FIELD OF THE INVENTION

Methods described herein relate to genomic analysis in general, and more specifically to the use of genomic information for detecting and treating cancer.

### BACKGROUND OF THE INVENTION

### Tumor genomics as predictor of anticancer treatment responses

Beyond inherent germline mutations, cancer cells often carry somatic gross chromosomal aberrations such as copy number variations, duplications or deletions of certain alleles or genomic regions. Some of these variants may thus cause a loss of some genomic functions such as for instance the tumor suppressor mechanisms, in particular the homologous recombination repair (HRR or HR) function, thus making the cancer more aggressive. Identifying those genomic mutations is of key importance in recent advances of personalized cancer medication, as they have been shown to be predictive of the response of a subject with a cell hyperproliferative disorder to certain anti-cancer therapies. The subject may be a human or an animal. Examples of anti-cancer treatments comprise such alkylating agents such as for instance, but not limited to, platinum-based chemotherapeutic agents, carboplatin, cisplatin, iproplatin, nedaplatin, oxaliplatin, picoplatin, chlormethine, chlorambucil, melphalan, cyclophosphamide, ifosfamide, estramustine, carmustine, lomustine, fotemustine, streptozocin, busulfan, pipobroman, procarbazine, dacarbazine, thiotepa, temozolomide and/or other antineoplastic platinum coordination compounds; a DNA damaging agent or radiation; an anthracycline, such as for instance, but not limited to, epirubincin or doxorubicin; a topoisomerase I inhibitor, such as for instance, but not limited to, campothecin, topotecan, irinotecan; and/or a PARP (poly ADP-ribose polymerase) inhibitor. Examples of a PARP inhibitors that exploits the tumor DNA homologous repair deficiency (HRD) to selectively destroy cancer cells are olaparib, rucaparib, niraparib, and talazoparib which have been approved in the US and in Europe for certain cancer types; other examples of PARP inhibitors include iniparib, CEP 9722, MK 4827, BMN-673, 3- aminobenzamide or velapirib. PARP inhibitors indirectly induce a multitude of DNA double strand breaks (DSBs) that prevents the development of HRD tumor cells while normal cells are generally able to repair those breaks via HR

### HRD genomic analysis tests

Accordingly, a method of classifying DNA samples, such as samples from a tumor, may therefore facilitate the diagnosis of possible cancer types or may allow to adapt the most suitable cancer treatment to patients in accordance with the characterization of gross DNA copy number aberrations in their tumor samples thanks to genomic sequencing and analysis. In particular, identification of whether a cancer is homologous repair deficiency (HRD) may be of considerable assistance in the planning of treatment. In the past decade, different genomic mutational signatures have been identified to this end. European patent EP2609216B1 discloses the use of a global chromosome aberration score (GCAS) to predict the outcome of an anti-cancer treatment such as a PARP inhibitor, radiation therapy or a chemotherapy comprising platinum-based chemotherapeutic agents. European patent EP2817630B1 proposes the detection of the number of telomeric allelic imbalance (TAI) events and the selection of a platinum-comprising therapy if this number is above a reference value from similar cancers known to be platinum-resistant. European patent EP2859118B1 by Stern, Manie and Popova from Institut Curie and INSERM discloses a method to predict HRD by counting the number, per genome, of segments spanning at least 3 megabases, which corresponds to large-scale transitions (LST) in chromosomal copy number. European patent EP2794907B1 by Abkevich et al. from Myriad Genetics discloses counting the total number of loss-of-heterozygosity (LOH) regions that are longer than 11 megabases but shorter than the whole chromosome in at least one pair of human chromosomes and comparing this number to a reference number to predict the patient response to a diverse possible cancer treatments; EP2981624B1 discloses using the LOH, TAI and LST indicators and EP3180447B1 discloses summing their values into an HRD score to be compared to a reference number in order to predict the patient response to a diverse possible cancer treatments. The latter test method is currently used as a test for HRD in ovarian cancer patients by the Myriad Genetics myChoice CDx assay, which uses a custom hybridization-based target-enrichment on a number of genes at a median coverage of at least 500X. This HRD score was also recently shown as a possible prognosis predictor regarding the progression of high-grade serous ovarian carcinoma (HGSOC) (Takaya et al, "Homologous recombination deficiency status-based classification of high-grade serous ovarian carcinoma", Nature Research Scientific Reports (2020) 10:2757).

Similarly to the Myriad myChoice assay, the Foundation Medicine commercial assay FoundationFocus CDx_{BRCALOH} specializes on the analysis of the BRCA1 and BRCA2 genes to determine whether an ovarian cancer patient will be responsive to responsive to rucaparib, a PARP inhibitor therapy. The latter assay also uses a custom hybridization-based capture of all coding exons of a number of genes at a median coverage of 500X, yet solely uses an LOH score assessed from the combination of a genome-wide copy number profile and minor allele fractions of SNPs.

### HRD analysis methods for WGS data

More generally, WO2017191074 proposes to characterize the HRD status of a tumor DNA sample according to a probabilistic score calculated from the analysis of different genomic alterations, including base substitutions, rearrangements and indel signatures (HRDetect score). As described in *"*HRDetect is a predictor of BRCA1 and BRCA2 deficiency based on mutational signatures", H. Davies et al., Nature Medicine, published online March 13th, 2017*,* with the latter HRDetect score, Whole Genome Sequencing (WGS) is shown as a possible method to detect HRD, with a 98.7% sensitivity, from mutational signatures observed throughout the whole genome. In contrast to WGS, when only Whole Exome Sequencing (WES) is applied, the sensitivity of HRDetect significantly drops, between 46.8% and 73% depending on specific adaptations of the bioinformatics algorithms. Beyond breast and ovarian cancer, as reported in "Genomic aberration based molecular signatures efficiently characterize homologous recombination deficiency in prostate cancer", Sztupinszki et al. also recently started to investigate the use of the LOH, TAI and LST as indicators of HRD signatures n prostate tumors out of WGS and WES data (scarHRD).

Since all the above methods rely on SNV and/or INDEL calling, they require a high depth of coverage (typically at least 30x) in next generation sequencing (NGS) workflows. High coverage requirements in WGS and large panels significantly increases the cost of the analysis in clinical practice, both in terms of wet lab experiments and dry lab data processing overhead. Alternative methods using low-pass WGS (LP-WGS - 1x to 5x) or even ultra-low-pass WGS (ULP-WGS - down to 0.1x) may thus be more advantageous for clinical oncology. In "ShallowHRD: Detection of Homologous Recombination Deficiency from shallow Whole Genome Sequencing", Bioinformatics, Apr 21 2020, Eeckhoutte et al. describe ShallowHRD, a software method to characterize the LST status similarly to the method of Manie, Stern and Pova, yet specifically on shallow WGS data, down to a coverage of about IX. Their approach simply uses the count of intra-chromosome arm copy number alterations. This count may be estimated as the number of large-scale transitions between adjacent segments of at least 10 megabases after removing segments of less than 3 Mb in the coverage data signal. According to the authors, less than 15 transitions enables to identify HRD negative tumors while more than 19 enables to identify HRD positive tumors with similar sensitivity and specificity as the prior art scarHRD method. While this method offers the promise for the use of more cost-effective NGS workflows in clinical oncology, we observe that its sensitivity however still remains lower than the results achieved by HRDetect and the measurement of the LST indicator on SNP arrays. This may be due to the fact that it cannot integrate the LOH and TAI signatures of the prior art methods. Moreover, it only enables to classify tumors for which the chromosomal alterations are large and frequent enough to cause more than a dozen large scale transitions, regardless of how those transitions are distributed within each chromosome arm on the one hand and among the multiple chromosomes of the human genome on the other hand.

A number of recent research works in oncology have suggested that the hypermutability of dysfunctional DNA may cause heterogenous alterations in different areas of the chromosomes. In *"*Regulation of mitotic recombination between DNA repeats in centromeres", Nucleic Acids Research, 2017, Vol. 45, No. 19, Zafar et al. In "The dark side of centromeres, types, causes and consequences of structural abnormalities implicating centromeric DNA", Barra et al., Nature Communications (2018) 9:4340, Barra et al. report that a significant ratio of chromosome rearrangements and breaks are observed in pericentromeric and centromeric regions in cancer cell lines derived from colorectal carcinomas and adenocarcinomas, probably due to an inherent fragility of the centromere regions in tumors. Barra et al. highlight the lack of models and technologies to explicitly analyze the highly repetitive sequences in those regions.

There is therefore a need for improved genomic analysis methods, which can be easily and cost-effectively deployed in automated NGS workflows for routine oncology clinical practice while being adaptive to recent cancer genomics discoveries and in particular to the promising discoveries of certain spatial features of chromosomal aberrations in the tumour genomes. Preferably, those methods and technologies may also be applicable at a lower coverage in order to decrease the overall analysis cost in routine clinical setups, while preserving a reasonable accuracy in the detection of HR events compared to prior art techniques. Moreover, improved genomic analysis methods may also facilitate the characterization of HR-deficient tumours by employing machine learning technologies instead of relying upon explicit measurement and thresholding of scalar HRD indicators such as the LOH, LST or TAI indicators of the prior art, so that they can more easily exploit the increasing clinical data availability in the classification of tumours for a diversity of different cancers in humans and animals.

### BRIEF SUMMARY

A method is proposed for characterizing a DNA sample, the method comprising obtaining a set of sequencing reads from a patient sample aligned to said reference genome; dividing the base pair positions (bp) for at least two chromosomes of a reference genome into a set of bins, each bin corresponding to a genomic region of at most 20 megabase pairs (20 Mbp), each bin solely containing coverage data from a single chromosome arm; estimating, from the aligned reads, the coverage data for each bin of the sequenced patient sample; arranging the coverage data into a multidimensional array comprising either the chromosomes or the chromosome arms along one dimension and the set of bins for said chromosome or chromosome arm along another dimension, characterized in that either the centromeric bins or the telomeric bins for each chromosome or chromosome arm are aligned into the multidimensional array spatial arrangement; inputting the multidimensional array to a trained machine learning model; and generating, at the output of the trained machine learning model, a chromosomal spatial instability (CSI) indicator to characterize the presence of a large scale genomic alteration in at least one region of at least one of the chromosome arms. In a possible embodiment, the CSI indicator may be an indicator of whether said patient sample has a high or low likelihood of being homologous recombination (HR)-deficient. The patient sample may be a tumor sample and the CSI indicator of the patient sample may be a predictor of the tumor response to a cancer treatment regimen comprising a platinum-based chemotherapeutic agent, a DNA damaging agent, an anthracycline, a topoisomerase I inhibitor, or a PARP inhibitor.

In a possible embodiment, the first set of bins may be further collapsed into larger bins prior to arranging the multidimensional array, by adapting the larger bin size with respect to each chromosome arm length such so that the bin size remains constant along the chromosome arm. The first set of bins may have a uniform size of at most 100kbp while the collapsed bins may have a size of at least 500kbp. The coverage data may be normalized and/or may be segmented to infer discrete copy number values prior to arranging the multidimensional array. In a possible embodiment, the empty elements of the multidimensional array may be padded with either the value of the closest bin or with a predefined value. In a possible embodiment, the trained machine learning model may be a random forest model or a neural network model, such as for instance a convolutional neural network trained to produce at its output a single label binary classification of the positive or negative CSI status, or a single label multiclass classification of the positive, negative or uncertain CSI status, or a scalar CSI score representative of the CSI status. The patient sample may be a tumor sample and the machine learning model may be trained in supervised or semi-supervised mode using a set of real samples labelled with an HRD status according to the HRDetect method and/or using artificial samples generated by sampling chromosomes of a set of real samples sharing the same HRD status and having similar tumor content. The artificial samples may be chosen in order to recreate the same purity-ploidy distribution as with the real samples dataset.

### BRIEF DESCRIPTION OF THE DRAWINGS

FIG.1 represents a next generation sequencing system according to certain embodiments of the present disclosure.
FIG.2 represents a genomic analysis workflow according to certain embodiments of the present disclosure.
FIG.3 schematically illustrates a possible embodiment of a low coverage data preparation pre-processing workflow comprising collapsing and smoothing of small coverage bins into larger bins to better evidence spatial events more specifically observable along distinct chromosome arms.
FIG.4 plots an example of a normalized read coverage data signal as may be initially measured with a first set of small bins from the coverage data signal of a low-pass whole genome sequencing experiment, along a set of 22 non sexual chromosomes in a human DNA sample.
FIG.5 plots an example of the normalized read coverage data signal as may be calculated with a second set of larger bins out from the coverage data signal FIG.4.
FIG.6 shows the whole set of the human genome chromosomes arranged so as to align their centromeric region, with their respective p-arm and q-arm chromosome arm lengths.
FIG.7 illustrates the normalized coverage data spatially re-arranged as a 2D matrix with chromosomes arranged per rows and the coverage bins arranged along the columns, such that the centromeric bins are vertically aligned in a single column, in accordance with certain embodiments of the proposed coverage data preparation methods, prior to chromosomal spatial instability analysis, respectively for a) an HRD negative tumor DNA sample and b) an HRD positive tumor DNA sample.
FIG.8 schematically represents the internal data workflow architecture of a possible chromosomal spatial instability analyzer.
FIG.9 shows a possible architecture of a convolutional neural network in accordance with certain embodiments of the proposed CSI Analyzer data processing module.
FIG.10a) and FIG. 10b) show the normalized coverage data for 2 samples of the 560 Breast Cancer genomic database.
FIG. 11 shows the normalized coverage data along the 22 non sexual chromosomes from the 202 BAM file samples from the 560 Breast Cancer genomic database.
FIG. 12 benchmarks the prior art HRD score method vs the proposed CSI indicator classifier against the HRDetect score.
FIG. 13 plots the HRD score, the proposed CSI indicator score and the HRDetect score results for a series of 69 test samples with different BRCA deficiency possibly in relation with HR deficiency.

### DETAILED DESCRIPTION

The present disclosure is based, at least in part, on the discovery that the presently disclosed machine learning trained analyzer, designed to process a tumor sample sequencing data coverage in relation with the underlying chromosomal arrangements, can extract an indicator of a chromosomal spatial instability (CSI) in general and in particular an indicator of a homologous repair deficiency (HRD) status for the tumor sample.

The proposed methods and systems will now be described by reference to more detailed embodiments. The proposed methods and systems may, however, be embodied in different forms and should not be construed as limited to the embodiments set forth herein. Rather, these embodiments are provided so that this disclosure will be thorough and complete, and will fully convey the scope to those skilled in the art.

### Definitions

A **"DNA sample"** refers to a nucleic acid sample derived from an organism, as may be extracted for instance from a solid tumor or a fluid. The organism may be a human, an animal, a plant, fungi, or a microorganism. The nucleic acids may be found in a solid sample such as a Formalin-Fixed Paraffin-Embedded (FFPE) sample. Alternately, the nucleic acids may be found in limited quantity or low concentration, such as circulating tumor DNA in blood or plasma.

A **"DNA fragment"** refers to a short piece of DNA resulting from the fragmentation of high molecular weight DNA. Fragmentation may have occurred naturally in the sample organism, or may have been produced artificially from a DNA fragmenting method applied to a DNA sample, for instance by mechanical shearing, sonification, enzymatic fragmentation and other methods. After fragmentation, the DNA pieces may be end repaired to ensure that each molecule possesses blunt ends. To improve ligation efficiency, an adenine may be added to each of the 3' blunt ends of the fragmented DNA, enabling DNA fragments to be ligated to adaptors with complementary dT-overhangs.

A **"DNA product"** refers to an engineered piece of DNA resulting from manipulating, extending, ligating, duplicating, amplifying, copying, editing and/or cutting a DNA fragment to adapt it to a next-generation sequencing workflow.

A **"DNA-adaptor product"** refers to a DNA product resulting from ligating a DNA fragment with a DNA adaptor to make it compatible with a next-generation sequencing workflow.

A **"DNA library"** refers to a collection of DNA products or DNA-adaptor products to adapt DNA fragments for compatibility with a next-generation sequencing workflow.

A **"pool"** refers to multiple DNA samples (for instance, 48 samples, 96 samples, or more) derived from the same or different organisms, as may be multiplexed into a single high-throughput sequencing analysis. Each sample may be identified in the pool by a unique sample barcode.

A **"nucleotide sequence"** or a **"polynucleotide sequence"** refers to any polymer or oligomer of nucleotides such as cytosine (represented by the C letter in the sequence string), thymine (represented by the T letter in the sequence string), adenine (represented by the A letter in the sequence string), guanine (represented by the G letter in the sequence string) and uracil (represented by the U letter in the sequence string). It may be DNA or RNA, or a combination thereof. It may be found permanently or temporarily in a single-stranded or a double-stranded shape. Unless otherwise indicated, nucleic acids sequences are written left to right in 5' to 3' orientation.

**"Amplification"** refers to a polynucleotide amplification reaction to produce multiple polynucleotide sequences replicated from one or more parent sequences. Amplification may be produced by various methods, for instance a polymerase chain reaction (PCR), a linear polymerase chain reaction, a nucleic acid sequence-based amplification, rolling circle amplification, and other methods.

**"Sequencing"** refers to reading a sequence of nucleotides as a string. High throughput sequencing (HTS) or next-generation-sequencing (NGS) refers to real time sequencing of multiple sequences in parallel, typically between 50 and a few thousand base pairs per sequence. Exemplary NGS technologies include those from Illumina, Ion Torrent Systems, Oxford Nanopore Technologies, Complete Genomics, Pacific Biosciences, BGI, and others . Depending on the actual technology, NGS sequencing may require sample preparation with sequencing adaptors or primers to facilitate further sequencing steps, as well as amplification steps so that multiple instances of a single parent molecule are sequenced, for instance with PCR amplification prior to delivery to flow cell in the case of sequencing by synthesis.

**"Aligning"** or **"alignment"** or **"aligner"** refers to mapping and aligning base-by-base, in a bioinformatics workflow, the sequencing reads to a reference genome sequence, depending on the application. For instance, in a targeted enrichment application where the sequencing reads are expected to map to a specific targeted genomic region in accordance with the hybrid capture probes used in the experimental amplification process, the alignment may be specifically searched relative to the corresponding sequence, defined by genomic coordinates such as the chromosome number, the start position and the end position in a reference genome. As known in bioinformatics practice, in some embodiments "alignment" methods as employed herein may also comprise certain pre-processing steps to facilitate the mapping of the sequencing reads and/or to remove irrelevant data from the reads, for instance by removing non-paired reads, and/or by trimming the adapter sequence as the end of the reads, and/or other read pre-processing filtering means.

**"Coverage"** refers to the number of sequencing reads that have been aligned to a genomic position or to a set of genomic positions. In general, a genomic region with a higher coverage is associated with a higher reliability in downstream genomic characterization, in particular when calling variants. In target enrichment workflows, only a small subset of regions of interest in the whole genome is sequenced and it may therefore be reasonable to increase the sequencing depth without facing too significant data storage and data processing overhead. In some genomic analysis applications not requiring a high resolution along the genome, for instance in detecting copy number alterations, low-pass (LP) coverage (1x-10x) or even ultra-low-pass (ULP) coverage (<1X - not all positions are sequenced) may be more efficient in terms of information technology infrastructure costs, but these workflows require more sophisticated bioinformatics methods and techniques to process the less reliable data input out from the sequencer and aligner. Moreover, apart from the higher cost related to data storage and processing, the operational cost of an experimental NGS run, that is, loading a sequencer with samples for sequencing, also needs to be optimized by balancing the coverage depth and the number of samples which may be assayed in parallel in routine clinical workflows. Indeed, next generation sequencers are still limited in the total number of reads that they can produce in a single experiment (i.e. in a given run). The lower the coverage, the fewer reads per sample for the genomic analysis, and the higher the number of samples that can be multiplexed within a next generation sequencing run.

A **"Bin",** a **"genomic section",** a **"partition",** a **"genomic portion"** or a **"portion of a chromosome"** refers to a contiguous region of interest of a genome. As such a region may include variants, a bin generally refers to the location or region of the genome rather than to a fixed DNA sequence. In bioinformatics methods and processes, a bin may be identified by its start and end genomic coordinates along the reference genome, and the bin length may be measured as a number of bases (b, kb, Mb) or basepairs (bp, kbp, Mbp) from the start to the end coordinates. In general, a bin may correspond to an entire chromosome, a segment of a chromosome, a segment of a reference genome, multiple chromosome portions, multiple chromosomes, portions from multiple chromosomes, and/or combinations thereof. Preferably, a bin is a portion of a chromosome obtained from partitioning of a reference genome (e.g., partitioned by size, segments, contiguous regions, contiguous regions of an arbitrarily defined size, and the like). Genomic sections may be selected, sorted, filtered and/or removed from consideration using any suitable criteria know in the bioinformatics. Along a genome, the bins can have the same, uniform length or different, variable lengths.

### "Count

**s"** refer to a number of sequencing reads or pair-end reads (representing a DNA fragment) that are mapped to a bin or partly overlap a bin. A count may be derived from some or all of the raw sequence reads and/or pre-processed sequence reads mapped to (i.e., associated with) a genomic section. Some of the sets of reads may be weighted, removed, filtered, normalized, adjusted, collapsed, merged, added, and/or subtracted, or processed by a combination thereof prior to counting them, in units of reads or in units of pair-end reads, in accordance with various bioinformatics methods known in the art. In some embodiments, a count may be associated with an uncertainty or a quality value. In some embodiments, a read or pair-end read may not be entirely contained in a single bin, but rather overlap over two adjacent bins; in this case the read may be counted within the bin with the largest overlap.

A **"coverage data signal"** refers to a collection of bins associated with their respective counts, which may be arranged as a 1D vector, a 2D matrix, or any suitable topology.

A **"chromosome arm"** refers to any of the two sections (arms) of a chromosome which are bound to each other by the chromosome centromere. The p-arm refers to the shortest arm while the q-arm refers to the longest arm. Each arm terminates with a telomere. In a metacentric chromosome, the p-arm and the q-arm have similar sizes. In a sub-metacentric chromosome, the p-arm is shorter than the q-arm. In an acrocentric chromosome, the p-arm is very short. In a telocentric chromosome, the p-arm no longer exists or is so short that it is no longer visible when examining the chromosome. Normal cells in humans do not carry telocentric chromosomes; however, they may be found in certain tumor cells.

A **"centromeric region"** for a chromosome refers to a range of positions corresponding to the centromere of a chromosome within a reference genomic coordinate system. Exemplary bioinformatics data representations with different coordinate systems (absolute or relative position indexing, 0-based or 1-based, etc) include the BED format, the GTF format, the GFF format, the SAM format, the BAM format, the VCF format, the BCF format, the Wiggle format, the GenomicRanges format, the BLAST format, the GenBank/EMBL Feature Table format, and others.

A **"centromeric bin"** for a chromosome coverage data signal refers to the coverage bin which is the closest to the centromeric region within the set of coverage bins associated with a chromosome arm. One or two centromeric bins may be associated with the genomic coverage data for any given human chromosome, one on the left of the centromere, one on the right of the centromere along the sequencing data genomic coordinate system.

A **"telomeric region"** for a chromosome refers to a range of positions mapping corresponding to the telomere of a chromosome within a given genomic coordinate. Exemplary bioinformatics data representations with different coordinate systems (absolute or relative position indexing, 0-based or 1-based, etc) include the BED format, the GTF format, the GFF format, the SAM format, the BAM format, the VCF format, the BCF format, the Wiggle format, the GenomicRanges format, the BLAST format, the GenBank/EMBL Feature Table format, and others.

A **"telomeric bin"** for a chromosome coverage data signal refers to the coverage bin which is the closest to any of the two telomeric regions respectively at the start and at the end of the chromosome within the set of coverage bins associated with this chromosome. Two separate, distant, telomeric bins may be associated with the genomic coverage data for any given human chromosome, one for the p-arm, the other for the q-arm.

A **"chromosomal instability"** or "CIN" refers to a type of genomic instability in which chromosomes are unstable, such that either whole chromosomes or parts of chromosomes are duplicated or deleted. More specifically, CIN refers to the increase in rate of addition or loss of entire chromosomes or sections of them. Such genomic alterations may occur in particular in tumor cells, involving either a gain or loss of whole chromosomes or structural aberrations, such as gross chromosomal rearrangements.

A **"chromosomal spatial instability"** or "CSI" refers to a chromosomal instability in which the increase in rate of addition or loss may be characterized according to a spatial distribution of events along the genome.

A **"machine learning model"** refers to a data model or a data classifier which has been trained using a supervised, semi-supervised or unsupervised learning technique as known in the data science art, as opposed to an explicit statistical model. The data input may be represented as a 1D signal (vector), a 2D signal (matrix), or more generally a multidimensional array signal (for instance a tensor, or a RGB color image represented as 3^{∗}2D signals of its Red, Green and Blue color decomposition planes - 3 matrices), and/or a combination thereof. In the case of a **deep learning classifier,** the data input is further processed through a series of data processing layers to implicitly capture the hidden data structures, the data signatures and underlying patterns. Thanks to the use of multiple data processing layers, deep learning facilitates the generalization of automated data processing to a diversity of complex pattern detection and data analysis tasks. The machine learning model may be trained within a supervised, semi-supervised or unsupervised learning framework. Within a supervised learning framework, a model learns a function to map an output result from an input data set, based on example pairs of inputs and matching outputs. Examples of machine learning models used for **supervised learning** include Support Vector Machines (SVM), regression analysis, linear regression, logistic regression, naive Bayes, linear discriminant analysis, decision trees, k-nearest neighbor algorithms, random forest, artificial neural networks (ANN) such as convolutional neural networks (CNN), recurrent neural networks (RNN), fully-connected neural networks, long short-term memory memory (LSTM) models, and others; and/or a combination thereof. A model trained within an unsupervised learning framework infers a function that identifies the hidden structure of a data set, without requiring prior knowledge on the data. Examples of **unsupervised machine learning models** known in the art include clustering such as k-means clustering, mixture model clustering, hierarchical clustering; anomaly detection methods; principal component analysis (PCA), independent component analysis (ICA), T-distributed Stochastic Neighbor Embedding (t-SNE); generative models; and/or unsupervised neural networks; autoencoders; and/or a combination thereof. **Semi-supervised learning** is a machine learning framework within which one can train a model using both labeled and unlabeled data. Unlabeled data, when used in conjunction with a small amount of labeled data, can produce considerable improvement in learning accuracy compared to other frameworks. This approach is particularly interesting when only part of the available data is labeled.

A **"convolutional neural network"** or **"CNN"** refers to a machine learning model which uses multiple data processing layers, known as convolutional layers, to represent the input data in a way which is best suited to solve a classification or regression task. During training, weight parameters are optimized for each CNN layer using optimization algorithms known in the art such as the backpropagation algorithm to perform a stochastic gradient descent. At runtime, the resulting trained CNN may then process very efficiently the input data, for instance to classify it into the right data output labels with as little false positives and false negatives as possible in the case of a learnt classification task. Convolutional neural networks may also be combined with recurrent neural networks to produce a deep learning classifier.

### Genomic analysis system

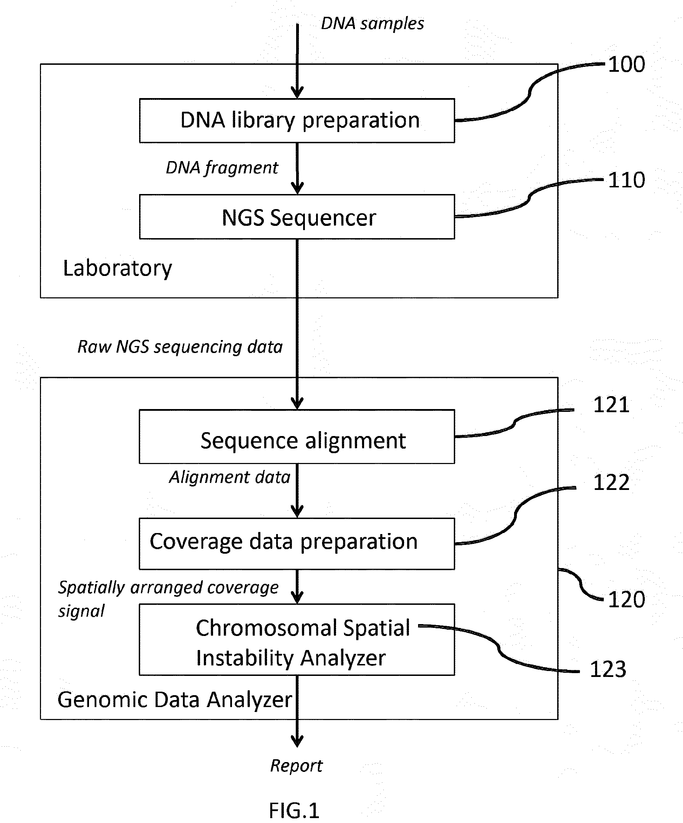

An exemplary genomic analysis system and workflow will now be described with further detail with reference to FIG. 1. As will be apparent to those skilled in the art of DNA analysis, a genomic analysis workflow comprises preliminary experimental steps to be conducted in a laboratory (also known as the "wet lab") to produce DNA analysis data, such as raw sequencing reads in a next-generation sequencing workflow, as well as subsequent data processing steps to be conducted on the DNA analysis data to further identify information of interest to the end users, such as the detailed identification of DNA variants and related annotations, with a bioinformatics system (also known as the "dry lab"). Depending on the actual application, laboratory setup and bioinformatics platforms, various embodiments of a DNA analysis workflow are possible. FIG. 1 describes an example of an NGS system comprising a wet lab system wherein DNA samples are first experimentally prepared with a DNA library preparation protocol 100 which may produce, adapt for sequencing and amplify DNA fragments to facilitate the processing by an NGS sequencer 110. In a next generation sequencing workflow, the resulting DNA analysis data may be produced as a data file of raw sequencing reads in the FASTQ format. The workflow may then further comprise a dry lab Genomic Data Analyzer system 120 which takes as input the raw sequencing reads for a pool of DNA samples prepared according to the proposed methods, and applies a series of data processing steps to characterize certain genomic features of the input samples. An exemplary Genomic Data Analyzer system 120 is the Sophia Data Driven Medicine platform (Sophia DDM) as already used by more than 1000 hospitals worldwide in 2020 to automatically identify and characterize genomic variants and report them to the end user, but other systems may be used as well. Different detailed possible embodiments of data processing steps as may be applied by the Genomic Data Analyzer system 120 for genomic variant analysis are described for instance in the international PCT patent application WO2017/220508, but other embodiments are also possible.

As illustrated on FIG. 1, the Genomic Data Analyzer 120 may comprise a sequence alignment module 121, which compares the raw NGS sequencing data to a reference genome, for instance the human genome in medical applications, or an animal genome in veterinary applications. In a conventional Genomic Data Analyzer system, the resulting alignment data may be further filtered and analyzed by a variant calling module (not represented) to retrieve variant information such as SNP and INDEL polymorphisms. The variant calling module may be configured to execute different variant calling algorithms. The resulting detected variant information may then be output by the Genomic Data Analyzer module 120 as a genomic variant report for further processing by the end user, for instance with a visualization tool, and/or by a further variant annotation processing module (not represented). In a preferred embodiment, the Genomic Data Analyzer system 120 may comprise automated data processing modules such as a coverage data preparation module 122 to prepare a spatially arranged coverage data signal, and a Chromosomal Spatial Instability (CSI) Analyzer module 123 to analyze the spatially arranged coverage data signal and derive CSI information, which may then be reported to the end user, for instance with a visualization tool, or to another downstream process (not represented).

### Data processing workflow

The Genomic Data Analyzer 120 may process the sequencing data to produce a genomic data analysis report by employing and combining different data processing methods.

The sequence alignment module 121 may be configured to execute different alignment algorithms. Standard raw data alignment algorithms such as Bowtie2 or BWA that have been optimized for fast processing of numerous genomic data sequencing reads may be used, but other embodiments are also possible. The alignment results may be represented as one or several files in BAM or SAM format, as known to those skilled in the bioinformatics art, but other formats may also be used, for instance compressed formats or formats optimized for order-preserving encryption, depending on the genomic data analyzer 120 requirements for storage optimization and/or genomic data privacy enforcement.

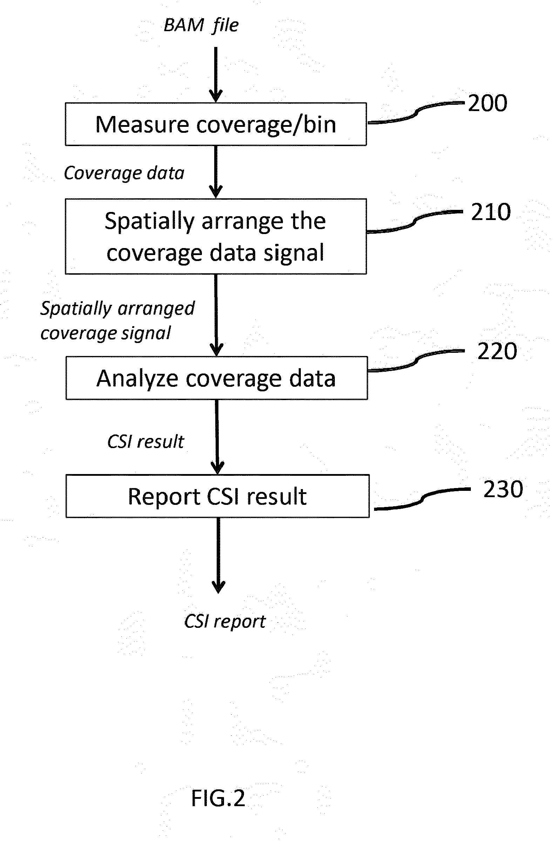

FIG.2 shows a possible workflow for the automated data processing modules to produce a CSI report from the analysis of an alignment file such as a BAM file in a bioinformatics pipeline. In a possible embodiment, the coverage data preparation module 122 may be adapted to measure 200 the coverage data signal from the aligned reads as the raw number of reads or pair-end reads aligned to each genomic bin and to arrange 210 the resulting data as a spatially arranged coverage data signal. The spatially arranged coverage data may be represented as a matrix, resp. as a set of matrices, or as a coverage multidimensional array data (tensor) to facilitate its processing by a CSI analyzer 123 operating on 2D image, resp. on a multiplane 2D image, or on a multidimensional array (tensor) input signal. The Chromosomal Spatial Instability (CSI) Analyzer module 123 may then further analyze, classify and/or categorize 220 the spatially arranged coverage data information to report 230 to the end user whether the data exhibits certain properties which characterize a possible chromosomal spatial instability (CSI) for the tumor sample. In a preferred embodiment, the CSI may characterize the homologous repair deficiency (HRD) of the tumor sample, but other genomic features in relation with a chromosomal spatial instability may also be characterized, for instance HRD caused by a BRCA1 or a BRCA2 deficiency. This report may then facilitate the oncogenomics diagnosis of the cancer subtype and/or its prognosis. This report may also facilitate the selection of a medical treatment specifically targeting the analyzed tumor, such as for instance the use of certain cancer treatments which exploits the deficiency of the HRR pathway to decrease or stop the tumor cellular multiplication.

The Genomic Data Analyzer 120 may be a computer system or part of a computer system including a central processing unit (CPU, "processor" or "computer processor" herein), memory such as RAM and storage units such as a hard disk, and communication interfaces to communicate with other computer systems through a communication network, for instance the internet or a local network. Examples of genomic data analyzer computing systems, environments, and/or configurations include, but are not limited to, personal computer systems, server computer systems, thin clients, thick clients, handheld or laptop devices, multiprocessor systems, microprocessor-based systems, set top boxes, programmable consumer electronics, network PCs, minicomputer systems, mainframe computer systems, graphical processing units (GPU), and the like. In some embodiments, the computer system may comprise one or more computer servers, which are operational with numerous other general purpose or special purpose computing systems and may enable distributed computing, such as cloud computing, for instance in a genomic data farm. In some embodiments, the genomic data analyzer 120 may be integrated into a massively parallel system. In some embodiments, the genomic data analyzer 120 may be directly integrated into a next generation sequencing system.

The Genomic Data Analyzer 120 computer system may be adapted in the general context of computer system-executable instructions, such as program modules, being executed by a computer system. Generally, program modules may include routines, programs, objects, components, logic, data structures, and so on that perform particular tasks or implement particular abstract data types. As is well known to those skilled in the art of computer programming, program modules may use native operating system and/or file system functions, standalone applications; browser or application plugins, applets, etc.; commercial or open source libraries and/or library tools as may be programmed in Python, Biopython, C/C++, or other programming languages; custom scripts, such as Perl or Bioperl scripts.

Instructions may be executed in distributed cloud computing environments where tasks are performed by remote processing devices that are linked through a communications network. In a distributed cloud-computing environment, program modules may be located in both local and remote computer system storage media including memory storage devices.

### Coverage data preparation - binning

In a preferred embodiment, the reference genome is first divided into a set of P bins and the number of DNA fragments mapping to each bin is counted taking as input the BAM or SAM file, using bioinformatics methods as known in the art, to produce 200 the coverage data signal along the P bins. As will be apparent to those skilled in the art of bioinformatics, in some embodiments, some of the bins with may be ignored, filtered, or merged with other bins to improve the input data quality. The counts for each bin along the genome, arranged as a raw coverage data signal.

In a possible simple embodiment, a uniform size may be used for the bins along the reference genome, but a choice of heterogeneous sizes is also possible. The size of the bins may be chosen such that it preserves enough spatial detail for the analysis of the coverage data signal for each chromosome while facilitating the coverage data signal analysis with the machine learning analysis methods described herein. Preferably, the size of the bins may be in the range from 300bp to 20Mpb, but other embodiments are also possible.

In the case of the reference human genome, chromosomes are sequentially ordered from the largest chromosome (chrl) to the smallest one (chr22), possibly with the exclusion of the sexual chromosomes. Human chromosomes are numbered by decreasing length, from 249Mbp for chrl down to 51Mbp for chr22. By dividing the reference genome in bins of 3Mbp, it is therefore possible to obtain a 1D array (vector) of 84 bins for the longest chromosome 1 down to 4 bins for the shortest chromosome 22. *Table 1* shows the theoretical respective start and end position of the centromeric region, the total chromosome length and its type, as well as the respective lengths in Mbp for the p-arm (short arm) and the q-arm (long arm) for each chromosome in the human genome, with reference to the GRCh37 reference genome (hs37d5 version), in the coordinate system of the reference genome used for the read alignment. Note that not all bins of the human genome may contain aligned reads in the BAM file with the current high throughput sequencing technologies, due to the difficulty to accurately mapping the short NGS reads to certain repetitive sequences or problematic regions of the human genome, for instance the p-arm of the acrocentric chromosomes 13, 14, 15, 21, 22 or Y.

**Table 1. Start and end positions of centromeric region positions, chromosome lengths (in bp) and chromosome arm lengths (in Mbp) for the human chromosomes expressed in the GRCh37 reference genome coordinate system (hs37d5 version)**

| CHR | START | END | CHRLEN | P-ARM (Mbp) | Q-ARM (Mbp) | TYPE |
|---|---|---|---|---|---|---|
| 1 | 121535434 | 124535434 | 249250621 | 124.715187 | **121.535434** | metacentric |
| 2 | 92326171 | 95326171 | 243199373 | **147.873202** | 92.326171 | submetacentric |
| 3 | 90504854 | 93504854 | 198022430 | 104.517576 | 90.504854 | metacentric |
| 4 | 49660117 | 52660117 | 191154276 | 138.494159 | 49.660117 | submetacentric |
| 5 | 46405641 | 49405641 | 180915260 | 131.509619 | 46.405641 | submetacentric |
| 6 | 58830166 | 61830166 | 171115067 | 109.284901 | 58.830166 | submetacentric |
| 7 | 58054331 | 61054331 | 159138663 | 98.084332 | 58.054331 | submetacentric |
| 8 | 43838887 | 46838887 | 146364022 | 99.525135 | 43.838887 | submetacentric |
| 9 | 47367679 | 50367679 | 141213431 | 90.845752 | 47.367679 | submetacentric |
| 10 | 39254935 | 42254935 | 135534747 | 93.279812 | 39.254935 | submetacentric |
| 11 | 51644205 | 54644205 | 135006516 | 80.362311 | 51.644205 | submetacentric |
| 12 | 34856694 | 37856694 | 133851895 | 95.995201 | 34.856694 | submetacentric |
| 13 | 16000000 | 19000000 | 115169878 | 96.169878 | 16 | acrocentric |
| 14 | 16000000 | 19000000 | 107349540 | 88.34954 | 16 | acrocentric |
| 15 | 17000000 | 20000000 | 102531392 | 82.531392 | 17 | acrocentric |
| 16 | 35335801 | 38335801 | 90354753 | 52.018952 | 35.335801 | metacentric |
| 17 | 22263006 | 25263006 | 81195210 | 55.932204 | 22.263006 | submetacentric |
| 18 | 15460898 | 18460898 | 78077248 | 59.61635 | 15.460898 | submetacentric |
| 19 | 24681782 | 27681782 | 59128983 | 31.447201 | 24.681782 | metacentric |
| 20 | 26369569 | 29369569 | 63025520 | 33.655951 | 26.369569 | metacentric |
| 21 | 11288129 | 14288129 | 48129895 | 33.841766 | 11.288129 | acrocentric |
| 22 | 13000000 | 16000000 | 51304566 | 35.304566 | 13 | Acrocentric |
| X | 58632012 | 61632012 | 155270560 | 93.638548 | 58.632012 | submetacentric |
| Y | 10104553 | 13104553 | 59373566 | 46.269013 | 10.104553 | acrocentric |

As will be apparent to those skilled in the arts of bioinformatics, in a possible embodiment, the bin sizes and positions along the whole genome sequencing data may also be adapted to the actual coverage from the BAM file. In general, we may consider that each bin in the set of P bins may be defined by 2 values corresponding to the start and end positions of the bin {*bin_start, bin_end*}_{*1*<=*bin_index*<=*P*} in the reference coordinate system. The size of the bin may thus be variable as *bin_size*= *bin_end - bin_start.* In a possible embodiment, the size of the bin may be in the range from 0.5Mbp to 1.5Mbp, or from 1.5Mbp to 2.5Mbp, or from 2.5Mbp to 3.5Mbp, or from 3.5Mbp to 4.5Mbp, or from 4.5Mbp to 5.5Mbp, or from 5.5Mbp to 6.5Mbp, or from 6.5Mbp to 7.5Mbp, or from 7.5Mbp to 8.5Mbp, or from 8.5Mbp to 9.5Mbp. In another possible embodiment, the size of the bins may be selected such that a fixed number of bins may be obtained for each chromosome or for each chromosome arm. Other embodiments are also possible.

In a possible embodiment, a first size of 100kbp may be used to produce a first set of high resolution coverage bins. The resulting high resolution coverage data signal may be further collapsed into a second set of larger bins of 1 to 20Mbp, for instance by combining about 30 100kbp-large initial bins to produce a bin of a larger size, between 2.5Mbp and 3.5Mbp, thus reducing the dimensionality of the data for the downstream CSI analysis. Various methods known to those skilled in the art of bioinformatics may be used to this end, such as smoothing, using the median or the mean to reduce the dimensionality, pooling, sampling, and other methods. In a preferred embodiment, collapsing uniquely assigns each bin to a specific chromosome arm without mixing coverage data from different chromosome arms into the bin. The coverage data is then clearly separated from one chromosome arm to the next one, which may facilitate the analysis of chromosomal spatial instability events at the chromosome arm level.

In a possible embodiment, collapsing may comprise adapting the size of the collapsed bins to each chromosome arm length in order to reduce the border effect at the chromosome arm boundaries. For instance, in a situation where the chromosome arm length is not dividable by the initial bin size considered, the last collapsed bin obtained will usually be shorter than the other ones, causing a border effect (for instance the centromeric bin or the telomeric bins on any end of the chromosome arm may not comprise an entire area of 3Mbp, and may result in a distorted coverage for those areas while possibly key in analyzing CSI events). A strategy to reduce this effect is to adapt the binning size with respect to chromosome arm length such that the chromosome arm length considered is dividable by the binning size with a neglectable remainder. This ensures that all the collapsed bins over the chromosome arm considered will have the same actual size.

In a possible further embodiment, collapsing may comprise adapting the start or the end position, and thus the actual size, of the collapsed bins individually along a chromosome arm such that the sum of the variable sizes of the collapsed bins still equals the chromosome arm length while being more or less evenly distributed along the chromosome arm.

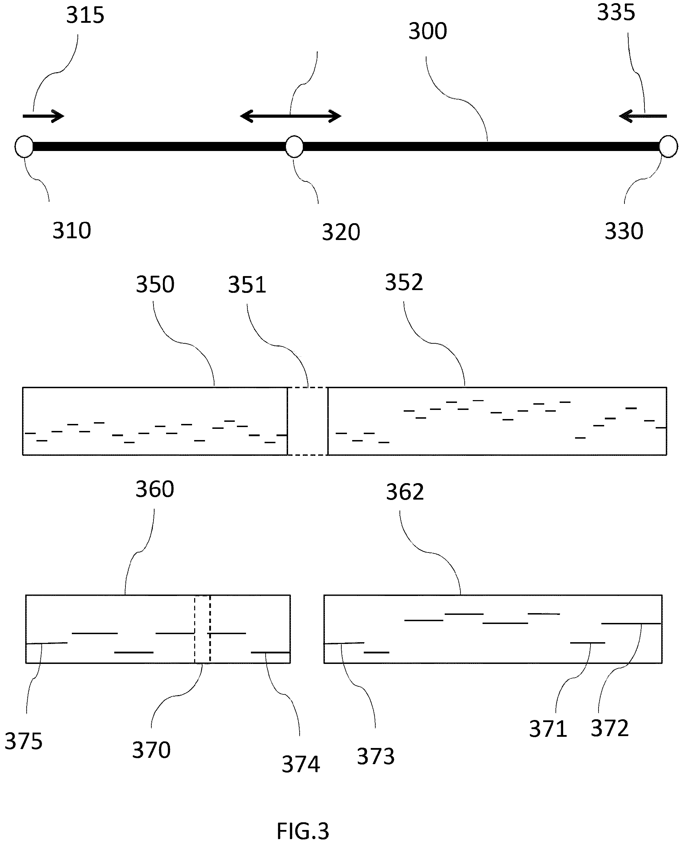

FIG.3 schematically illustrates a chromosome 300 comprising a centromeric region 320, a start position 310 corresponding to the p-arm telomere and an end position 340 corresponding to the q-arm telomere. The chromosome includes a first telomeric region 315 starting from the start position 310 (coordinate 0 in the BAM file coordinate format), a centromeric region 325 around the centromere (region between positions 121535434 and 124535434 in chrl in the BAM file coordinate system as listed in Table 1), and a second telomeric region 335 ending at the end position 330. As will be apparent to those skilled in the art of bioinformatics, most of the centromeric region 351 has no associated reference sequence within the existing reference genome covering this area. and thus cannot be mapped. Such a region may then be skipped during the binning process (in the case of chrl, this region would correspond to an entire 3Mbp bin). In a possible embodiment, the high-resolution bins corresponding to limited coverage areas or low quality areas may also be removed in accordance with genomic knowledge of the corresponding regions (e.g. region 355). In a preferred embodiment, a different set of first high resolution coverage bins 350, 352 may be separately constructed for each chromosome arm from the BAM file. Each set of high-resolution bins may then be further collapsed into a second set of larger scale bins 360, 362 for each arm. The bins may also span shorter or larger genomic regions (for instance, a bin 371 of 2.5Mbp followed by a bin 372 of 3.5Mbp still averaging to a 3Mbp binning) in accordance with genomic knowledge of the corresponding regions and/or to optimize binning at the respective ends of the two chromosome arms (telomeric bins 372, 375 or centromeric bins 373, 374).

For example, the initial 100kbp bins from each chromosome arm may be smoothed independently to obtain bins of 3Mbp (i.e aggregation over 30 bins of 100kbp). As the total number of bins in each chromosome arm may not be exactly dividable by 30, the last smoothed bin of chromosome arms usually contains less than 30 original bins. This may either give those regions too much importance if keeping them, or conversely if removing them, losing too much information. To reduce the border effect, larger bins of variable size may be collapsed for each chromosome arms independently. In a possible embodiment, the bin size that leads to the smallest non-complete bin remainder and that is closest to the target size may be selected. In a possible embodiment, the last bin if not complete may then be rejected as now neglectable, but other embodiments are also possible. Table 2 column 3 shows an exemplary distribution of the proposed collapsed bin sizes for each chromosome arm for an initial set of 100kbp bins collapsed into larger bins at a target 3Mbp size along the 2^{∗}22 chromosome arms. Column 4 shows the resulting sizes of the rejected border bins, compared to the default number, in column 2, of remainder higher resolution bins that would form the last collapsed bin at the arm border when dividing by a fixed size of 3Mpb for all arms.

**Table 2. Exemplary bin collapsing distribution, from 100kbp bins into a target collapsed bin size of 3MBp. Coverage data preparation - normalization**

| **Chr arm** | **Remainder bins** | **Collapsed bin size (Mbp)** | **Border bins size (kbp)** |
|---|---|---|---|
| Chr 1_p : | 2 | 3 | 200 |
| Chr 1_q : | 9 | 2.6 | 300 |
| Chr 2_p : | 25 | 2.6 | 300 |
| Chr 2_q : | 5 | 3.3 | 200 |
| Chr 3_p : | 21 | 2.6 | 300 |
| Chr 3_q : | 28 | 2.6 | 0 |
| Chr 4_p : | 4 | 2.5 | 400 |
| Chr 4_q : | 16 | 2.9 | 100 |
| Chr 5_p : | 7 | 2.5 | 200 |
| Chr 5_q : | 1 | 3 | 100 |
| Chr 6_p : | 20 | 3.3 | 200 |
| Chr 6_q : | 14 | 2.7 | 800 |
| Chr 7_p : | 21 | 3.3 | 300 |
| Chr 7_q : | 2 | 2.9 | 100 |
| Chr 8_p : | 4 | 2.8 | 200 |
| Chr 8_q : | 7 | 2.6 | 100 |
| Chr 9_p : | 5 | 2.6 | 100 |
| Chr 9_q : | 2 | 3 | 200 |
| Chr 10_p : | 18 | 2.9 | 0 |
| Chr 10_q : | 29 | 2.7 | 200 |
| Chr 11_p : | 12 | 3.3 | 0 |
| Chr 11_q : | 24 | 3.1 | 0 |
| Chr 12_p : | 20 | 3.2 | 0 |
| Chr 12_q : | 26 | 2.8 | 0 |
| Chr 13_q : | 7 | 2.5 | 700 |
| Chr 14_q : | 18 | 2.5 | 300 |
| Chr 15_q : | 2 | 3 | 200 |
| Chr 16_p : | 17 | 3.2 | 100 |
| Chr 16_q : | 25 | 3.4 | 700 |
| Chr 17_p : | 8 | 3.1 | 200 |
| Chr 17_q : | 25 | 2.8 | 100 |
| Chr 18_p : | 14 | 3.3 | 200 |
| Chr 18_q : | 6 | 3.2 | 0 |
| Chr 19_p : | 9 | 2.7 | 0 |
| Chr 19_q : | 25 | 3.3 | 100 |
| Chr 20_p : | 10 | 2.5 | 0 |
| Chr 20_q : | 24 | 2.7 | 0 |
| Chr 21_q : | 7 | 3.4 | 100 |
| Chr 22_q : | 27 | 2.7 | 0 |

As will be apparent to those skilled in the art of bioinformatics, in some embodiments, the raw coverage data signal may be further normalized, filtered or smoothed by a method known in the art. In a possible embodiment, normalization may comprise normalizing per sample, by dividing the coverage data signal by the mean coverage signal for the whole sample, in order to get rid of biases associated with the sample sequencing experiment possibly causing differences in the coverage per sample. Normalization may also comprise normalization by GC content to apply a GC-bias correction. Other embodiments are also possible, such as bin-wise normalization, linear and nonlinear least squares regression, GC LOESS (GC normalization), LOWESS, PERUN (normalization per sample), RM, GCRM, cQn and/or combinations thereof.

In a possible further embodiment, the normalized data may be further segmented into sub-regions within the bins to identify homogeneous areas within the coverage data signal, possibly corresponding to different copy numbers (normally 2 per chromosome). Coverage segmentation allows to infer discrete copy number segments from the coverage profile of a given sample. To do so, the coverage profile is decomposed into segments (i.e portion of the genome) within which the coverage is believed to be constant. Each of the segments defined in the previous step may then be associated to discrete levels defined in terms of coverage signal amplitude. At this stage, Copy Number Variation (CNV) events can already be detected but the absolute Copy Number associated to each coverage level remains unknown.

In cases where the sample tumor content and ploidy is known or can be inferred from the data, the discrete levels identified by segmentation can be mapped to integer numbers reflecting the number of copies present in the tumor (e.g, CN=1, CN=2, ...). As will be apparent to those skilled in the art of bioinformatics, such segmentation methods with or without an absolute copy number reference may reduce the noise and facilitate the identification of the copy number profile for the sample. However, in the presence of noise, segmentation may produce erroneous results by suppressing coverage fluctuations which results from changes in copy number, in particular when sequencing at low coverage depths. In a preferred embodiment the genomic analysis methods as described herein may therefore operate without the segmentation step. In an alternate embodiment, a segmentation step may be applied to pre-process the data prior to applying the machine learning methods as disclosed herein. After possible steps of normalization and/or segmentation, the coverage data generally consists of a set of 1D vectors, each of them corresponding to a chromosome. In a possible embodiment, the size of the vectors may be variable, reflecting the fact that different chromosomes have different sizes. In another possible embodiment, the bins have a variable size so that each chromosome vector has the same size.

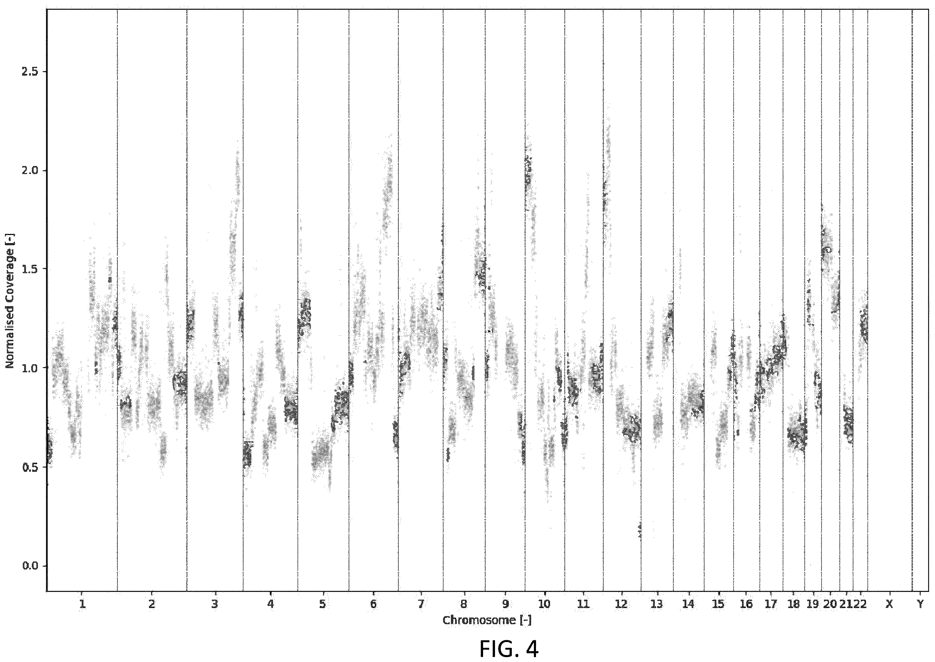

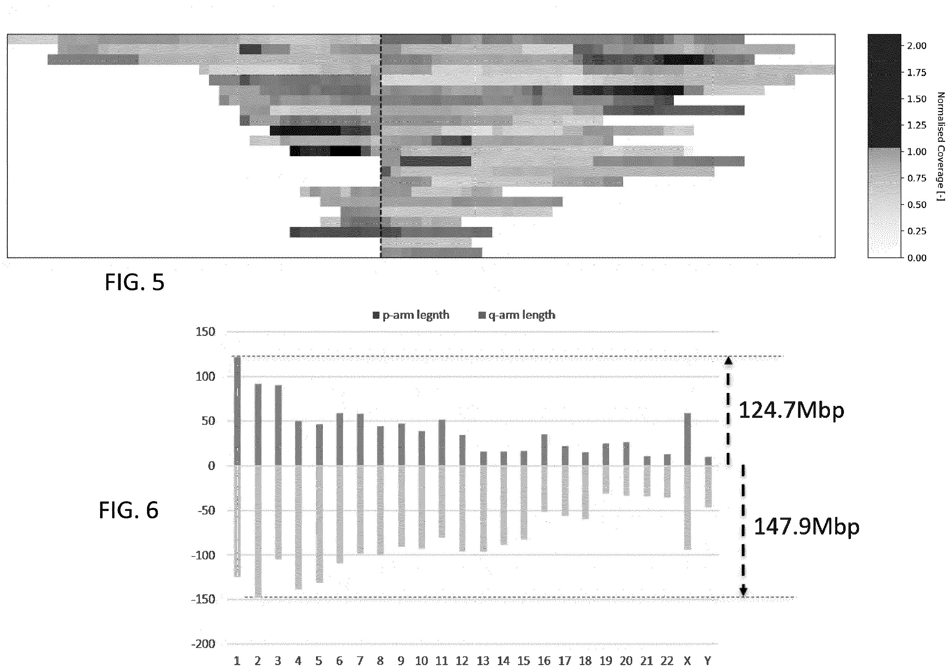

An exemplary coverage data 1D signal is plotted on FIG.4 for a whole genome NGS sequencing experiment at IX coverage over the first 22 chromosomes of the human genome (excluding the sexual chromosomes), with 100kbp bins, after normalization by sample and by GC-content. FIG. 5 shows the same coverage data signal arranged per chromosome with collapsed bins of 3Mbp.

### Spatial arrangement of the coverage data

In a preferred embodiment, the coverage data may therefore be further spatially arranged 210 to form a multi-dimensional data array in which the coverage data is organized to align the bins for each chromosome or chromosome arm along any of their telomeric or centromeric bins in the array structure. A multidimensional array structure may be particularly well suited to benefit from efficient genomic analysis machine learning trained models recently developed for non-genomic applications such as image classifiers, instead of relying upon simple human engineered features as in the prior art HRD detection methods.

FIG.6 shows a graphical representation of the chromosomes aligned according to their centromere position. As can be seen in Table 1 and FIG.6, the longest chromosome chrl is 249Mbp long, the longest p-arm is 124.7Mbp long for chrl, while the longest q-arm is actually longer at 147.9Mbp in chr2. In a possible embodiment with a given constant bin size *bin size,* the coverage data may be fit in a (*ceiling(chr_p-arm_max_len)*/*bin_size* + ceiling(*chr_q-arm_max_len)*/*bin_size*) ^{∗} *N* two-dimensional array, where N is the number of chromosomes to represent, *chr_p-arm_max_len* is the longest p-arm length and *chr_q-arm_max_len* is the longest q-arm length in the set of *N* chromosomes to be jointly analyzed. As an illustrative example, for the whole genome sequencing coverage data over the arms of the 22 non sexual chromosomes aligned by their centromeric bins, with bins at a constant size of *bin_size* =3Mbp, an array of dimension 92^{∗}22 (or 22^{∗}92) may be spatially arranged 210 by the Genomic Data Analyzer 120 as the data input to the CSI Analyzer 123. The coverage data for the longest p-arm of 124.7Mbp (chrl) may indeed be fit into 42 bins of 3Mbp while the longest q-arm of 147.9Mbp (chr2) may be fit into 50 bins of 3Mbp. In a possible embodiment the multidimensional array may be arranged to represent the 2^{∗}*N* chromosome arms over the bins for the longest chromosome arm in the set of N chromosomes to be analyzed. For example, the coverage data may be spatially arranged as an array of dimension 44^{∗}50 for analyzing the 22 chromosomes at a constant bin size of 3Mbp. The coverage data for each chromosome arm may be arranged as a row (or as a column) in the array so that the chromosome arms centromeric regions are aligned in the array. In a possible embodiment, the adaptive binning schematically shown on FIG.3 may be applied to remove irrelevant high-resolution bins and to adjust variable bin sizes and positions along the chromosome genomes. For instance, an array of dimension 84^{∗}22 (or 22^{∗}84) may be spatially arranged 210 by the Genomic Data Analyzer 120 as the data input to the CSI Analyzer 123. Other embodiments are also possible.

In a possible embodiment, the chromosome arms may be represented as rows in the array and the centromeric bins for all arms may be aligned along the first column of the array. In another possible embodiment, the chromosome arms may be represented as rows in the array and the telomeric bins for all arms may be aligned along the first column of the array. In another possible embodiment, the chromosome arms may be represented as rows in the array and the centromeric bins for all arms may be aligned along the last column of the array. In another possible embodiment, the chromosome arms may be represented as rows in the array and the telomeric bins for all arms may be aligned along the last column of the array.

In a possible embodiment, the chromosome arms may be represented as columns in the array and the centromeric bins for all arms may be aligned along the first row of the array. In another possible embodiment, the chromosome arms may be represented as columns in the array and the telomeric bins for all arms may be aligned along the first row of the array. In another possible embodiment, the chromosome arms may be represented as columns in the array and the centromeric bins for all arms may be aligned along the last row of the array. In another possible embodiment, the chromosome arms may be represented as columns in the array and the telomeric bins for all arms may be aligned along the last row of the array.

In a possible embodiment, the whole chromosomes may be represented as columns in the array and the centromeric bins for all chromosomes may be aligned along a row of the array. The alignment row for the centromeric bins may be at the center of the array or it may be shifted up or down from the center according to the respective bin lengths for the p-arms and the q-arms of the set of chromosomes. The bins associated with the p-arm and the bins associated with the q-arm for a chromosome may be on above and below respectively from the centromeric bin row, or conversely below and above from the centromeric bin row.

In a possible embodiment, the whole chromosomes may be represented as rows in the array and the centromeric bins for all chromosomes may be aligned along a column of the array. The alignment column for the centromeric bins may be at the middle of the array or it may be shifted right or left from the middle according to the respective bin lengths for the p-arms and the q-arms of the set of chromosomes. The bins associated with the p-arm and the bins associated with the q-arm for a chromosome may be right and left respectively from the centromeric bin column, or conversely left and right from the centromeric bin column.

In a possible embodiment, the whole chromosomes may be represented as rows in the array and one of the two telomeric bins for all chromosomes may be aligned along a column of the array. In a possible embodiment, the alignment column for the telomeric bins may be the first column of the array. In another possible embodiment, the alignment column for the telomeric bins may be the last column of the array. In a possible embodiment, the p-arm telomeric bins may be used for the alignment. In another possible embodiment, the q-arm telomeric bins may be used for the alignment. In another possible embodiment, the choice of the p-arm or the q-arm telomeric bin for the alignment may be selected individually for each chromosome.

In a possible embodiment, the whole chromosomes may be represented as columns in the array and one of the two telomeric bins for all chromosomes may be aligned along a row of the array. In a possible embodiment, the alignment row for the telomeric bins may be the first row of the array. In another possible embodiment, the alignment row for the telomeric bins may be the last row of the array. In a possible embodiment, the p-arm telomeric bins may be used for the alignment. In another possible embodiment, the q-arm telomeric bins may be used for the alignment. In another possible embodiment, the choice of the p-arm or the q-arm telomeric bin for the alignment may be selected individually for each chromosome.

As the number of bins changes with the length of the chromosome and/or the chromosome arms, some elements of the array need to be filled in by padding virtual data not available from the coverage data. As will be apparent to those skilled in the art of data science, different options are possible to pad the empty entries (or elements) of the multidimensional array. In a possible embodiment, the empty elements may be filled by a constant value. In another possible embodiment, the empty elements may be filled from a mask array of predefined constant values. In another possible embodiment, the empty elements may be filled as a function of the coverage data in the part of the row or the column which has been filled by real data. In a possible embodiment, the empty elements may be padded by repeating the coverage value left or right (resp. above or below) from the last available bin in the column (resp. row) of bins along a chromosome or chromosome arm row (resp. column).

In another possible embodiment, the individual size of the bins may be selected so as to facilitate a denser filling of the array. Smaller bin sizes (corresponding to a better resolution) may be selected in areas of particular genomic interest to increase the total number of bins for some chromosome arms.

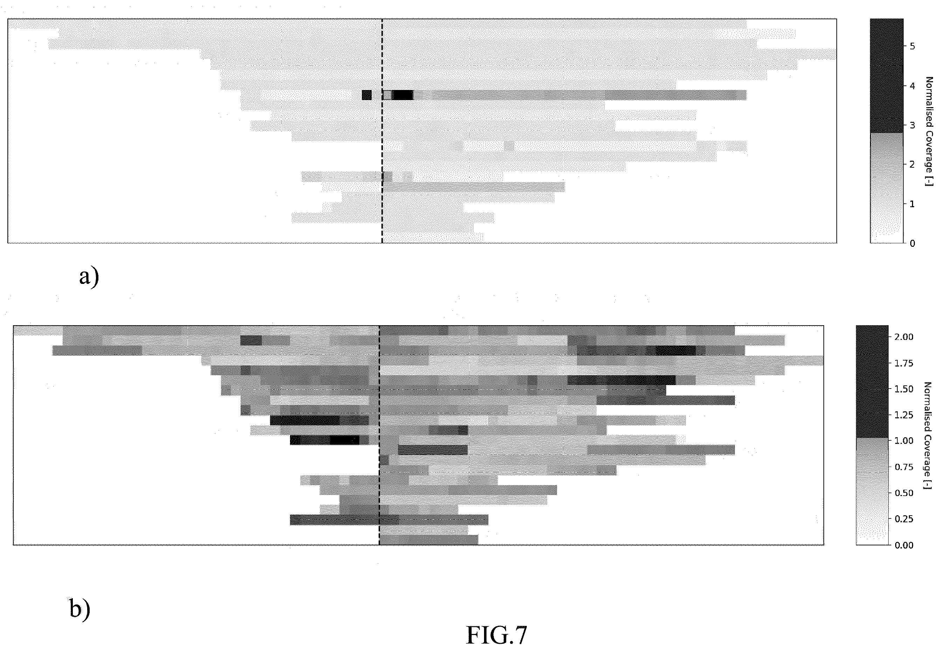

FIG.7 shows an example of the normalized coverage data spatially re-arranged as a 2D array image suitable for CSI analysis with a machine learning model, respectively from a) an HRD negative tumor DNA sample and b) an HRD positive tumor DNA sample. Chromosomes are arranged per rows and the coverage bins arranged along the columns, such that the centromeric bins are vertically aligned in a single column, in accordance with certain embodiments of the proposed coverage data preparation methods, prior to chromosomal spatial instability analysis. The brighter the pixel, the higher the normalized coverage count value *C_{bin}* for the plotted bin, and conversely the darker the pixel the lower the value.

### Chromosomal Spatial Instability (CSI) Analyzer

The Genomic Data Analyzer system 250 may further apply a chromosomal spatial instability analyzer 123 to the spatially arranged coverage data multi-dimensional array to automatically analyze and report one or more genomic properties characterizing certain spatial instability patterns for two of more chromosomes or chromosome arms in each DNA sample. In the case of a tumor sample analysis, the CSI analyzer module 123 may report the burden of chromosomal aberrations such as large deletions or duplications of an allele in a given genomic region along each chromosome arm. In a possible embodiment, in the case of a tumor sample, the CSI analyzer module 123 may identify a signature -which may identify samples that share a similar features and may thus respond well to a given treatment.

The CSI analyzer module 123 may accordingly identify, categorize and report an indicator, a scalar score or a combination of feature indicators to characterize one or more genomic properties of the tumor sample. In a preferred embodiment, the CSI analyzer module 123 may accordingly identify, categorize and report that the tumor sample is HRD-negative or HRD-positive, or may report a scalar score as an indicator of the HRD likelihood for the tumor sample. As will be apparent to those skilled in the art, this bioinformatics method then significantly facilitates detailed understanding of the cancer cells genomic alterations in the tumor sample and the adaptation of a personalized medicine treatment to the specifics of the inferred cancer cell biology for the patient.

In one embodiment, the genomic property report may be displayed to the end user on a graphical user interface. In another possible embodiment, the genomic property report may be produced as a text file for further automated processing. Other embodiments are also possible.

In a preferred embodiment, the CSI analyzer module 123 may be adapted to apply a machine learning model to the spatially arranged coverage data multi-dimensional array. In a possible embodiment, the CSI analyzer module 123 may comprise a trained neural network or a combination of trained neural networks as a data processing module to analyze 220 the spatially arranged coverage data multi-dimensional array as the trained neural network input and to produce a CSI result as the trained neural network output.

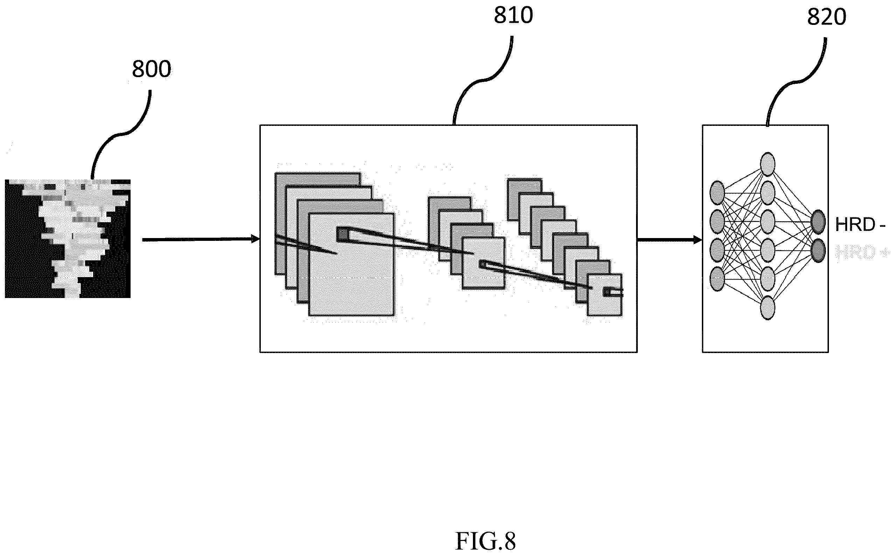

An exemplary CSI Analyzer 123 and its data processing workflow will now be described with further detail with reference to FIG.8. As will be apparent to those skilled in the art of machine learning, different methods and architectures known in the art may be used to process the prepared coverage data multidimensional array input and to derive a CSI status or a CSI score for the sample.

### Exemplary CNN machine learning model

In order to classify a multidimensional array such as a 2D image in which chromosome arms have been aligned along their respective centromeric positions to highlight visual patterns which characterize the CSI status of this chromosome set, a convolutional neural network (CNN) may be architected and trained by employing methods and technologies known in the art of image pattern recognition such as TensorFlow, Caffe, Caffe2, Pytorch, Theano, or others.

FIG. 8 schematically represents an example of a convolutional neural network (CNN) which may process the spatially arranged coverage data matrix image for a patient tumor DNA sample to classify it as either HRD positive (HRD+) or HRD negative (HRD-). In the example of FIG.8, the trained CNN takes as input a spatially arranged coverage data matrix image 800 plotted with a row arrangement of the 22 non sexual chromosomes, with chr 1 at the top down to chr 22 at the bottom, and a column arrangement of the bins (in the exemplary illustration of FIG.8, at around a 3Mbp size, aligned along the centromeric region of the chromosomes, with a basic padding at 0 coverage count, but other embodiments are possible too). The input image 800 is input to a first convolutional neural network 810 with several processing layers, to quantify a set of intermediary features which is itself input to a second neural network classifier 820 in charge with extracting two scores, one for HRD-, one for HRD+, as its two outputs. The CSI analyzer may then derive an HRD status (HRD-, HRD+, or uncertain) by comparing and thresholding the HRD- and HRD+ output values.

More generally, as will be apparent to those skilled in the art of deep learning, a CSI Analyzer 123 may be adapted to employ various CNN architectures. The convolutional network may comprise a first series of serially connected convolutional data processing layers 810, which may further feed a second series of fully connected layers 720 to output various predictions. The CNN architecture may for instance comprise one or more convolutional layers arranged to process ID, 2D or multidimensional data; optionally, one or more maximum-, median- or average-pooling layers arranged to process ID, 2D or multidimensional data; one or more multiple dropout layers, to facilitate regularization during training; optionally, one or more batch normalization layers; a flattening layer followed by one or more fully connected layers; or a combination thereof. Different activation methods may be used as known in the art, such as for instance ReLU, SELU, or more generally any activation function that prevents vanishing gradient. Different output activation may also be used as known in the art, for instance a sigmoid method, to produce the probability associated to a single label binary classification (for instance, probability of positive status, probability of negative status = 1 - probability of positive status), or a softmax method to produce the probabilities associated to a single label multiclass classification output (for instance, mutually exclusive probabilities of a positive status, negative status, or an uncertain status), or a regression method such a linear method or the ReLU classification method to produce a scalar value which is not meant to be interpreted as a probability, such as for instance the HRD score.

In a preferred embodiment, the CSI Analyzer 123 produces a final output decision such as an HRD+ or an HRD- status analysis for each sample, by thresholding the output from the CNN. The threshold may be adapted depending on the application, so as the optimize the sensitivity and/or the specificity of the CSI analysis according to the end user needs, for instance for diagnosis, prognosis, or to guide the choice of the most efficient cancer treatment. In a possible embodiment, the CSI Analyzer 123 may also further report 230 the CNN output value(s) in accordance with the end user needs.

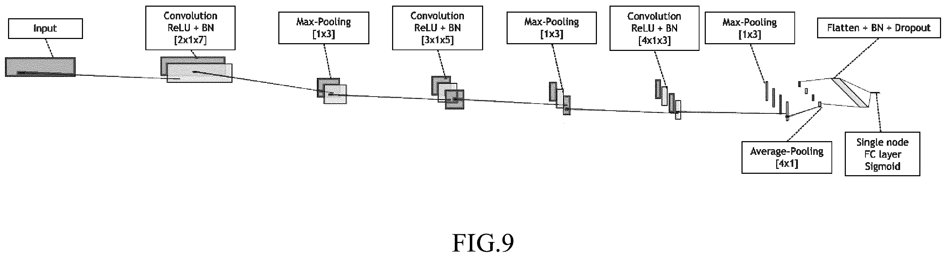

FIG. 9 shows an example of a possible detailed CNN architecture as may be employed by the CSI analyzer 123 for analyzing 220 the spatially arranged coverage data. For each convolution layer internal to the network, the number of filters as well as the filters dimension are represented as [N x H x W] where N is the number of filters, H is the filter height and W is the filter width. Each convolution is passed through an activation function (Rectified Linear Unit, ReLU) followed by Batch Normalization (BN). Intermediate convolutional layers may be interleaved with max-pooling layers, followed at the end by an average pooling and a flattening layer in accordance with usual CNN architectures. In this example, the output layer of the CNN is flattened, passed through a Batch Normalization layer, a Dropout layer, and given as input to a single node dense layer (i.e Fully Connected layer) using the sigmoid activation function.

### Chromosomal Spatial Instability (CSI) Analyzer training

For supervised and semi-supervised CSI Analyzer trainings, a set of labelled input-output pairs may be used as a training set. For instance, to train the CSI Analyzer to classify the HRD status, a subset of the public domain HRD labelled data samples such as those from may be used to enable the network to minimize its output errors by adjusting its parameters (e.g. weights) along its different layers through an optimization process such as back propagation which is specifically applied during the training phase. As will be apparent to those skilled in the art of machine learning, different loss functions may be used to measure the error, for instance a binary cross entropy measurement for a model with a single label binary classification output, a categorical cross entropy measurement for a model with a single label multiclass classification output, or a regression method based on the root mean square error, the absolute error, or other error measurements. Optimization may employ state of the art stochastic descent gradient based optimizers, such as ADAM or RMPSprop. Regularization may employ a dropout method, an early-stop method, and/or an L-1 or L-2 parameter regularization. Other embodiments are also possible.

As more clinical conditions linked to chromosomal instability get classified by current and future oncology research, additional datasets may be used for training machine learning models aimed at detecting genomic scars induced by HRD or other phenomena such a response to a given treatment. In a possible embodiment, the HRD+ and HRD- statuses as well as an "uncertain" status may used as multiclass ground truth labels. In another possible embodiment, the HRD score from a prior art method such as HRDetect may be used as a scalar output label. The input coverage data may also be spatially arranged as a multidimensional data according to any of the possible embodiments herein disclosed, as long as the choice of the multidimensional array format embodiment remains the same at training time and at runtime.

In a possible embodiment, a semi-supervised learning framework may be employed for training the machine learning model. Indeed, access to labeled data can be challenging and costly, particularly in the context of HRD status prediction which makes semi-supervised learning a promising framework for this application. Since the degree of model overfitting is determined by both its complexity and the amount of training it receives, providing a model with more training examples can help reduce overfitting. Data augmentation consists in the creation of new artificial training samples from existing data. In a possible embodiment, artificial samples may be generated by sampling chromosomes from available real samples sharing the same HRD status and having similar tumor content (or bionomically preprocessed to mimic similar tumor content) and ploidy.

### Experiments - example 1

In a first experiment, the CNN of FIG.9 has been trained using the low pass WGS coverage data measured in a subset of 133 tumor samples included in the 560 breast cancer patients, for which the BRCA-deficiency status and HRD status, as defined by the HRDetect score, is publicly available, with reference to Nik-Zainal et al., "Landscape of somatic mutations in 560 breast cancer whole-genome sequences", Nature 534, 47-54 (2016) - data set from the Wellcome Trust Sanger Institute and the International Cancer Genome Consortium ICGC available at the European Genome-phenome Archive EGA (https://www.ebi.ac.uk/ega/studies/EGAS00001001178) and to "HRDetect is a predictor of BRCA1 and BRCA2 deficiency based on mutational signatures", H. Davies et al., Nature Medicine, published online March 13th, 2017*.* This original training dataset of 133 publicly available real samples was augmented to 3083 artificial training samples. Data augmentation was performed to preserve the same purity-ploidy distribution observed in the original dataset. The artificial samples obtained via data augmentation were only used for training and validation. A significant portion of the original dataset (69 samples of the 202 taken from 560 Breast Cancer study) was kept for testing and was not involved in the data augmentation procedure.

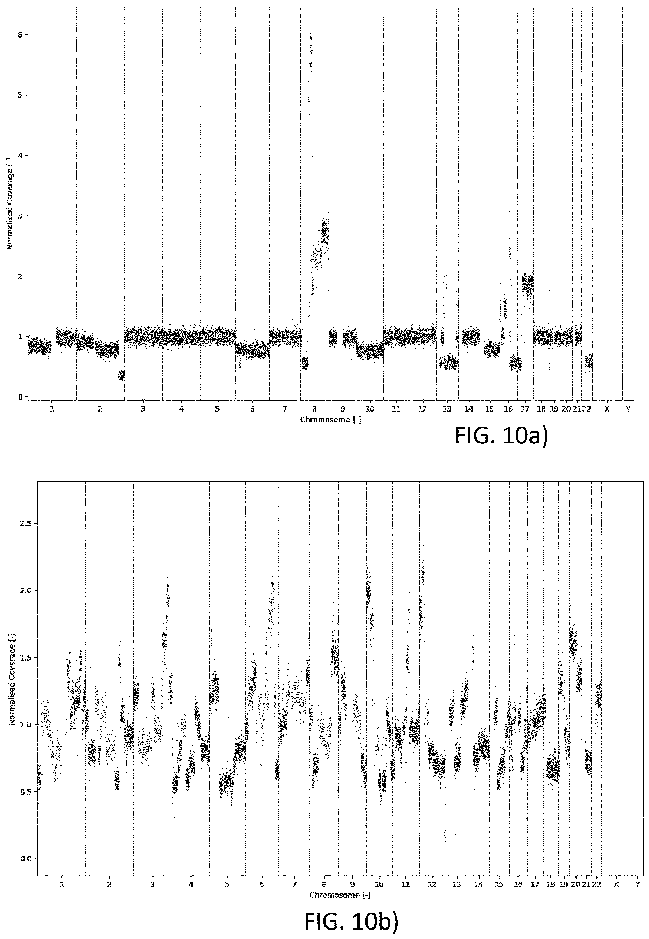

The original BAM file of each tumor sample was first downsampled to 10 million pair-end reads to mimic low-pass WGS, the coverage signal was first pre-computed using a first set of high-resolution coverage bins of 100kbp, and was preprocessed by performing sample and GC normalization. The resulting normalized coverage signal (y-axis) is displayed respectively on FIG.10a) for an HRD-sample and FIG.10b) for an HRD+ sample over the whole genome excluding sexual chromosome (x-axis). The x-axis gives the genomic coordinates with vertical dashed lines at the border of each chromosome.

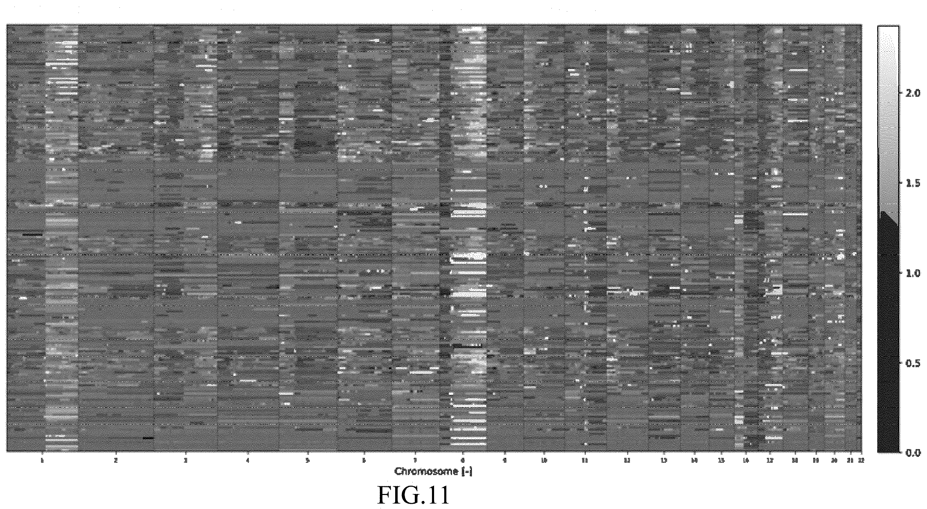

The normalized coverage data was further collapsed into an average 3Mbp target bin size in the 2.5Mpb-3.5Mbp range using the adaptive binning strategy of FIG.3. FIG.11 shows the resulting coverage data as 1D signals, one row per sample, prior to applying the spatial arrangement methods according to some embodiments of the present disclosure, for the 133 original training samples sorted by their CSI status, with HR-deficient samples on the top of the y-axis. The x-axis shows the genomic coordinates with vertical dashed lines at the border of each chromosome.

The normalized coverage data was further spatially arranged to form a 2D array of 84 bins * 22 chromosomes that can be provided as input to a CNN. The coverage bins for 22 chromosomes are plotted as 22 rows from chrl down to chr 22, aligned with respect to their centromeric bins. For shorter chromosome arms, empty bins are filled by copying the value of nearest telomeric bin present in the same row. FIG.7a) shows the resulting array for an HRD- sample while FIG.7b) shows the resulting array for an HRD+ sample.

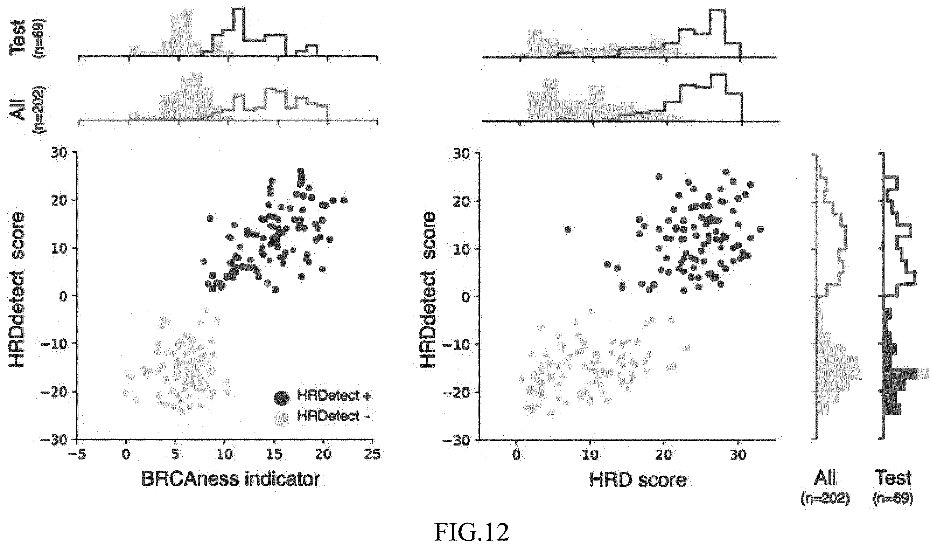

FIG. 12 shows the results obtained by applying the trained CNN on the full datataset of 202 samples comprising the 69 test samples not used for training (indicated as a "BRCAness" status indication as measured at the output of the CNN, prior to conversion into a probability via softmax), on the left, and the results of the prior art HRD score (obtained by averaging LOH, LST and TAI score), on the right, benchmarked as classifiers against the HRDetect score. As can be seen in the histograms shown in FIG. 12, the proposed method outperforms the prior art HRD score method at predicting the HRDetect positive samples, regardless of testing on the whole set of 202 real samples or solely on the set of 69 samples not used for training the machine learning model.

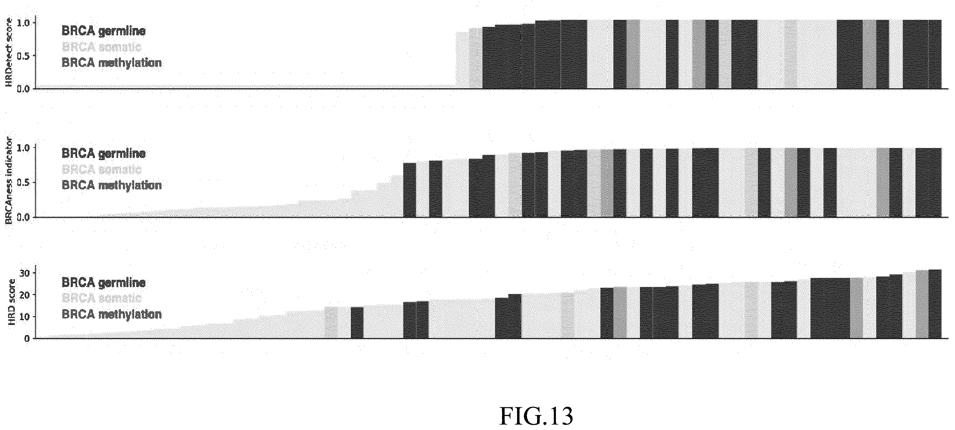

FIG. 13 shows the three HRDetect score (top), the HRD score (bottom) and the SOPHiA CSI (herein indicated as BRCAness) score obtained at the output of the trained CNN (middle), for each of the 69 samples in the test set. In each individual panel, the samples are sorted left to right by increasing score result. Samples without BRCA-deficiency are displayed in light gray. BRCA-deficient samples are shown in dark gray (different grey levels are used to denote the type of mutation causing BRCA-deficiency). As evidenced in FIG. 13, HRDetect score and SOPHiA BRCAness CSI score are very high for all BRCA-deficient sample. This result indicates that the CSI indicator outperforms the HRD score in identifying BRCA-deficient samples.

### Further advantages and benefits of the proposed methods

The proposed machine learning methods further bring additional advantages as they are inherently adaptive to the increasing availability of personalized medicine data, in particular in oncology practice. They do not require explicit biology models (for instance of suspected events occurring preferably in certain chromosome arm regions, such as around the telomeres or the centromeres) to establish predictive features suitable for guiding diagnosis, prognosis, and/or treatment. When employing a semi-supervised training framework, data augmentation facilitates the development of more robust data models less sensitive to noisy data or low tumor content in the DNA sample. As more data becomes available, the model may be retrained without the need to rearchitect the runtime genomic analyzer system and workflows, as only the model parameters change. Trained models initially developed for a specific application (e.g. breast cancer) may be transferred to other applications (e.g. ovarian cancer, prostate cancer or pancreatic cancer) and/or other sample types (FFPE, cfDNA). They may also be used to predict different statuses, including responses to different therapies and treatments.

### Other embodiments and applications

While various embodiments have been described above, it should be understood that they have been presented by way of example and not limitation. It will be apparent to persons skilled in the relevant art(s) that various changes in form and detail can be made therein without departing from the spirit and scope. In fact, after reading the above description, it will be apparent to one skilled in the relevant art(s) how to implement alternative embodiments.

While exemplary embodiments and applications of the proposed methods have been described in relation with the targeted next generation sequencing genomic analysis, it will be apparent to those skilled in the art of bioinformatics that they may also be adapted to apply to the detection and classification of HR deficiency out of alternate tumor sample genomic analysis workflows, for instance using genomic data out of SNP arrays and array CGH wet lab workflows. An SNP array, an array CGH, or a next-generation-sequencing (NGS) technology may be used to produce a count that varies with the copy number. Moreover, low pass WGS (0.1x to 5x) or large targeted panels such as for instance WES or CES panels may be solely used or combined to generate the input data to the proposed machine learning methods. Alternatively, low-pass WGS can be combined with small targeted panels (amplicon-based or capture-based) in a single or separate wetlab workflows. Possibly, off-target reads issued from targeted sequencing methods may also be used as main or complementary input data into the multidimensional array, while remaining well suited for a trained neural network as the input data processing component in the CSI Analyzer 123 architecture.

The Genomic Data Analyzer 120 computer system (also "system" herein) 120 may be programmed or otherwise configured to implement different genomic data analysis methods in addition to the CSI and HRD analyzer systems and methods as described herein, such as receiving and/or combining sequencing data, calling copy number alterations and/or annotating variants to further characterize the tumor samples. The machine learning model may employ extended multidimensional array inputs further comprising information such as GC content, bin size, mappability, mapping quality, variant allele fraction (VAF) in addition to the normalized coverage data 2D array input. The machine learning model may also employ additional scalar inputs providing tumor content information (purity), sample ploidy information. The Genomic Data Analyzer system may also be used to assess the quality of a sample (i.e. measure the degree of FFPE degradation).

While exemplary embodiments and applications of the proposed methods have been described in relation with a CNN as the trained machine learning model, in a possible embodiment (not illustrated), a random forest machine learning model may be alternately employed. Random forests or random decision forests are an ensemble learning method for classification, regression and other tasks that operate by constructing a multitude of decision trees at training time and outputting the class that is the mode of the classes (classification) or mean prediction (regression) of the individual trees. Random decision forests correct for decision trees' habit of overfitting to their training set which makes it a good candidate for high dimensional input data.

Unless otherwise defined, all technical and scientific terms used herein have the same meaning as commonly understood by one of ordinary skill in the art to which this invention belongs. The terminology used in the description herein is for describing particular embodiments only and is not intended to be limiting. As used in the description and the appended claims, the singular forms "a," "an," and "the" are intended to include the plural forms as well, unless the context clearly indicates otherwise.

Unless indicated to the contrary, the numerical parameters set forth in the following specification and attached claims are approximations that may vary depending upon the desired properties sought to be obtained and thus may be modified by the term "about". At the very least, and not as an attempt to limit the application of the doctrine of equivalents to the scope of the claims, each numerical parameter should be construed in light of the number of significant digits and ordinary rounding approaches.

Notwithstanding that the numerical ranges and parameters setting forth the broad scope are approximations, the numerical values set forth in the specific examples are reported as precisely as possible. Any numerical value, however, inherently contains certain errors necessarily resulting from the standard deviation found in their respective testing measurements. Every numerical range given throughout this specification will include every narrower numerical range that falls within such broader numerical range, as if such narrower numerical ranges were all expressly written herein.

As will be apparent to those skilled in the art of digital data communications, the methods described herein may be indifferently applied to various data structures such as data files or data streams. The terms "data", "data set", "data structures", "data fields", "file", or "stream" may thus be used indifferently throughout this specification.

Although the detailed description above contains many specific details, these should not be construed as limiting the scope of the embodiments but as merely providing illustrations of some of several embodiments.

In addition, it should be understood that any figures which highlight the functionality and advantages are presented for example purposes only. The disclosed methods are sufficiently flexible and configurable such that they may be utilized in ways other than that shown.

## Claims

1. A method of characterizing a patient DNA sample, the method including the steps of:
- Obtaining a set of sequencing reads from a patient DNA sample aligned to said reference genome;
- Dividing the base pair positions (bp) for at least two chromosomes of a reference genome into a set of bins, each bin corresponding to a genomic region of at most 20 megabase pairs (20 Mbp), each bin solely containing coverage data from a single chromosome arm;
- Estimating, from the aligned reads, the coverage data for each bin of the sequenced patient sample;
- Arranging the coverage data into a multidimensional array comprising either the chromosomes or the chromosome arms along one dimension and the set of bins for said chromosome or chromosome arm along another dimension, **characterized in that** either the centromeric bins or the telomeric bins for each chromosome or chromosome arm are aligned into the multidimensional array spatial arrangement;
- Inputting the multidimensional array to a trained machine learning model;
- Generating, at the output of the trained machine learning model, a chromosomal spatial instability (CSI) indicator to characterize the presence of a large scale genomic alteration in at least one region of at least one of the chromosome arms.

2. The method of claim 1, further comprising:
- based on said chromosomal spatial instability indicator, identifying whether said patient sample has a high or low likelihood of being homologous recombination (HR)-deficient.

3. The method of claim 1, further comprising a step of collapsing the bins into larger bins prior to arranging the multidimensional array, by adapting the bin size according to the chromosome arm length such that the bin size remains constant along each chromosome arm.

4. The method of claim 3, wherein the first set of bins have a uniform size of at most 100kbp and the collapsed larger bins have a size of at least 500kbp.

5. The method of any preceding claims, wherein the coverage data is normalized prior to arranging the multidimensional array.

6. The method of any preceding claims, wherein the coverage data is segmented to infer discrete copy number values prior to arranging the multidimensional array.

7. The method of any preceding claims, wherein the elements of the multidimensional array before the start of a chromosome arm or a chromosome which is shorter than the longest chromosome arm or chromosome in the array are padded with either the value of the first bin at the start of the chromosome arm or of the chromosome or with a predefined value, and wherein the elements of the multidimensional array beyond the end of a chromosome arm or a chromosome which is shorter than the longest chromosome arm or chromosome in the array are padded with the value of the last bin at the end of the chromosome arm or of the chromosome or with a predefined value.

8. The method of any preceding claims, wherein the trained machine learning model is a random forest model or a neural network model.

9. The method of claim 8, wherein the trained machine learning model is a convolutional neural network trained to produce at its output a single label binary classification of the positive or negative CSI status, or a single label multiclass classification of the positive, negative or uncertain CSI status, or a scalar CSI score representative of the CSI status.

10. The method of any preceding claims, wherein the machine learning model has been trained in supervised or semi-supervised mode using at least a set of real samples labelled with an HRD status according to the HRDetect method.

11. The method of any preceding claims, wherein the machine learning model has been trained in supervised or semi-supervised mode using artificial samples generated by sampling chromosomes of a set of real samples sharing the same HRD status and having similar patient content.

12. The method of claim 11, wherein the patient sample is a tumor sample and the artificial samples are chosen in order to recreate the same purity-ploidy distribution as with the real samples dataset.

13. An *in vitro* method of characterizing a DNA sample obtained from a patient, the method comprising:
- Isolating fragments of DNA from a patient sample;
- Constructing a library comprising said fragments overlapping a set of chromosomes;
- Sequencing the library to at most 30X genome wide sequencing coverage;
- Aligning the resulting sequencing reads to a reference genome for the patient sample;
- And generating, with the method of claim 1, a chromosomal spatial instability indicator for the patient sample.

14. The method of any preceding claims, wherein the patient sample is a tumor sample and the chromosomal spatial instability indicator of the patient sample is a predictor of the tumor response to a cancer treatment regimen comprising a platinum-based chemotherapeutic agent, a DNA damaging agent, an anthracycline, a topoisomerase I inhibitor, or a PARP inhibitor.

15. A PARP inhibitor for use in a method for treating cancer in a patient wherein said patient tumor sample has been predicted to have a HR deficiency according the method of claim 2.
